(19) Europäisches Patentamt
European Patent Office
Office européen des brevets

(11) **EP 4 667 448 A1**

(12) **EUROPEAN PATENT APPLICATION**
published in accordance with Art. 153(4) EPC

(43) Date of publication:
**24.12.2025 Bulletin 2025/52**

(21) Application number: **24757163.1**

(22) Date of filing: **13.02.2024**

(51) International Patent Classification (IPC):
*C07C 29/141* (2006.01)   *C07C 29/94* (2006.01)
*C07C 31/20* (2006.01)   *B01J 23/72* (2006.01)

(52) Cooperative Patent Classification (CPC):
(C-Sets available)
**B01J 23/72; B01J 12/007**   (Cont.)

(86) International application number:
**PCT/KR2024/001979**

(87) International publication number:
**WO 2024/172434 (22.08.2024 Gazette 2024/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.02.2023   KR 20230018881**
**08.02.2024   KR 20240019690**

(71) Applicant: **LG Chem, Ltd.**
**Yeongdeungpo-gu**
**Seoul 07336 (KR)**

(72) Inventors:
• **EOM, Sungshik**
**Daejeon 34122 (KR)**
• **KIM, Tae Yun**
**Daejeon 34122 (KR)**
• **SUH, Myungji**
**Daejeon 34122 (KR)**
• **YANG, Sungpil**
**Daejeon 34122 (KR)**
• **YANG, Jusang**
**Daejeon 34122 (KR)**
• **JUNG, Euiyoung**
**Daejeon 34122 (KR)**
• **CHA, Kyong Yong**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD FOR PREPARING NEOPENTYL GLYCOL**

(57)    A method for preparing neopentyl glycol, including packing an inlet of a hydrogenation reactor with a first hydrogenation catalyst including copper and having has a specific surface area of 200 m$^2$/g or more and a density of 500 g/L or more and less than 600 g/L; packing an outlet of the hydrogenation reactor with a second hydrogenation catalyst different from the first catalyst; introducing a raw material gas containing hydroxypivaldehyde and hydrogen into the hydrogenation reactor; and carrying out a hydrogenation reaction. The method for preparing neopentyl glycol can enhance the stability of the process for preparing neopentyl glycol and increase the yield of neopentyl glycol because the catalyst strength is excellent even under high-temperature and high-pressure conditions.

FIG. 2

(52)  Cooperative Patent Classification (CPC): (Cont.)

C-Sets
**C07C 29/141, C07C 31/20**

**Description**

This application is a National Stage Application of International Application No. PCT/KR2024/001979 filed on February 13, 2024, which claims priority to and the benefit of Korean Patent Application Nos. 10-2023-0018881 and 10-2024-0019690 filed in the Korean Intellectual Property Office on February 13, 2023 and February 8, 2024, respectively, the entire contents of which are incorporated herein by reference.

Technical Field

The present invention relates to a method for preparing neopentyl glycol.

**Background Art**

Neopentyl glycol (NPG) is a white crystalline material with a melting point of 130°C or higher and is used as an important intermediate for various synthetic resins, and is also widely used industrially as a raw material for various plastic powder coatings, synthetic lubricants, plasticizers, surfactants, fiber processing agents, and the like.
Such NPG is generally prepared by subjecting isobutyraldehyde and formaldehyde to an aldol condensation reaction to prepare hydroxypivaldehyde (HPA), and then reacting this HPA with hydrogen in the presence of a catalyst. In this case, in order to obtain a high yield, the reaction is carried out in the presence of a copper-based catalyst under high temperature (160°C or higher) and high pressure (35 bar or higher) conditions.
Since the reaction is carried out under such high temperature and high pressure conditions, a problem is that decreases in the strength of the catalyst may occur. When the strength of the catalyst decreases, the reactivity of the catalyst decreases, which may cause problems in terms of process stability.
Further, since the reaction is carried out under high temperature and high pressure conditions, a silica component, which is a catalyst support, is eluted, so that there may also occur a problem in that the silica component in a neopentyl glycol solution to be prepared is eluted. These problems may require additional processes to purify the silica component.
Therefore, continuous efforts are being made to develop a catalyst with improved catalyst stability and excellent catalyst activity due to excellent catalyst strength even under high temperature and high pressure conditions. In addition, continuous efforts are being made to develop technologies capable of preventing the elution of the catalyst component.
[Citation List] (Patent Document 1) Korean Patent Application Laid-Open No. 10-2011-0038324

**Brief Description**

**Technical Problem**

The present invention has been made in an effort to provide a method for preparing neopentyl glycol, which reduces the amount of catalyst component eluted while maintaining a high yield.

**Technical Solution**

An exemplary embodiment of the present invention provides a method for preparing neopentyl glycol, the method including: packing an inlet of a hydrogenation reactor with a first hydrogenation catalyst including copper; packing an outlet of the hydrogenation reactor with a second hydrogenation catalyst; introducing a raw material gas including hydroxypivaldehyde and hydrogen into the hydrogenation reactor packed with the first hydrogenation catalyst and the second hydrogenation catalyst; and carrying out a hydrogenation reaction while sequentially bringing the introduced raw material gas into contact with the first hydrogenation catalyst and the second hydrogenation catalyst, in which the first hydrogenation catalyst has a specific surface area of 200 $m^2$/g or more and a density of 500 g/L or more and less than 600 g/L, and the first hydrogenation catalyst and the second hydrogenation catalyst are different from each other and satisfy the following Equations 1 and 2.

$$[\text{Equation 1}]$$

$$x + y = 100(\%)$$

$$[Equation\ 2]$$

$$x > y$$

In Equations 1 and 2,

x means the packing proportion (%) of the first hydrogenation catalyst in the hydrogenation reactor, and
y means the packing proportion (%) of the second hydrogenation catalyst in the hydrogenation reactor.

An exemplary embodiment of the present invention provides a method for preparing neopentyl glycol, the method including: packing an inlet of a hydrogenation reactor with a first hydrogenation catalyst including copper; sequentially packing the hydrogenation reactor with a second hydrogenation catalyst and a third hydrogenation catalyst, such that an outlet of the hydrogenation reactor is packed with the third hydrogenation catalyst and a location between a location packed with the first hydrogenation catalyst and a location packed with the third hydrogenation catalyst is packed with the second hydrogenation catalyst; introducing a raw material gas including hydroxypivaldehyde and hydrogen into the hydrogenation reactor packed with the first hydrogenation catalyst to the third hydrogenation catalyst; and carrying out a hydrogenation reaction while sequentially bringing the introduced raw material gas into contact with the first hydrogenation catalyst to the third hydrogenation catalyst, in which the first hydrogenation catalyst to the third hydrogenation catalyst are different from each other and satisfy the following Equations 3 and 4.

$$[Equation\ 3]$$

$$x + y + z = 100(\%)$$

$$[Equation\ 4]$$

$$x > y + z$$

In Equations 3 and 4,

x means the packing proportion (%) of the first hydrogenation catalyst in the hydrogenation reactor,
y means the packing proportion (%) of the second hydrogenation catalyst in the hydrogenation reactor, and
z means the packing proportion (%) of the third hydrogenation catalyst in the hydrogenation reactor.

**Advantageous Effects**

The method for preparing neopentyl glycol according to the present invention can enhance the stability of the process for preparing neopentyl glycol by preventing the elution of catalyst components even in a high-temperature and high-pressure reaction. That is, since the process can be carried out for a long time, productivity can be increased.
The method for preparing neopentyl glycol according to the present invention can increase the yield of neopentyl glycol.

**Brief Description of Drawings**

FIG. 1 is a view illustrating an existing method for preparing neopentyl glycol.
FIGS. 2 and 3 are views illustrating the method for preparing neopentyl glycol according to the present invention.

[Explanation of Reference Numerals and Symbols]

1: Reaction raw material supply pipe
2: Reactor
3: Region packed with first hydrogenation catalyst

4: Region packed with second hydrogenation catalyst

4': Region packed with second hydrogenation catalyst and third hydrogenation catalyst

5: Reaction product outlet pipe

6: Reaction product circulation pipe

**Detailed Description**

Hereinafter, the present invention will be described in detail such that a person skilled in the art to which the present invention pertains can easily carry out the present invention. However, the present invention may be implemented in various different forms, and is not limited to the configurations described herein.

When one part "includes" one constituent element in the present specification, unless otherwise specifically described, this does not mean that another constituent element is excluded, but means that another constituent element may be further included.

In the present specification, 'p to q' means 'p or more and q or less'.

In the present specification, "specific surface area" is measured by the BET method, and is specifically calculated from an amount of nitrogen gas adsorbed under liquid nitrogen temperature (77K) using BELSORP-mino II manufactured by BEL Japan, Inc. That is, in the present invention, the BET specific surface area may mean a specific surface area measured by the measurement method.

In the present specification, the value of 'density' was determined by measuring the weight of the catalyst introduced into a 1 L measuring cylinder typically used in the art.

In the present specification, as 'diameter', a value obtained by measuring the diameters of 20 catalyst samples using vernier calipers typically used in the art, and then averaging the values was used.

In the present specification, 'nth hydrogenation catalyst' is an expression used to classify hydrogenation catalysts according to the packed location, and the hydrogenation catalyst that is packed first based on the inlet of a reactor is defined as a first hydrogenation catalyst. When a reactor is vertically oriented, the inlet of the reactor may be expressed as the top of the reactor, the outlet of the reactor may be expressed as the bottom of the reactor, and regardless of the direction of the reactor, the region between the inlet (or top) of the reactor and the outlet (or bottom) of the reactor may be expressed as the middle of the reactor.

Further, in describing the present invention, detailed descriptions of related known techniques that may unnecessarily obscure the gist of the present invention will be omitted.

An exemplary embodiment of the present invention provides a method for preparing neopentyl glycol, the method including: packing an inlet of a hydrogenation reactor with a first hydrogenation catalyst including copper; packing an outlet of the hydrogenation reactor with a second hydrogenation catalyst; introducing a raw material gas including hydroxypivaldehyde and hydrogen into the hydrogenation reactor packed with the first hydrogenation catalyst and the second hydrogenation catalyst; and carrying out a hydrogenation reaction while sequentially bringing the introduced raw material gas into contact with the first hydrogenation catalyst and the second hydrogenation catalyst, in which the first hydrogenation catalyst and the second hydrogenation catalyst are different from each other, and the first hydrogenation catalyst has a specific surface area of 200 $m^2/g$ or more and a density of 500 g/L or more and less than 600 g/L, and satisfies Equations 1 and 2 above.

The method for preparing neopentyl glycol according to the present invention is characterized by using a mixture of two or more hydrogenation catalysts which exhibit different core performance for the operating conditions according to the location of the reactor. In addition, the first hydrogenation catalyst inevitably includes copper, and is characterized in that the inside of the hydrogenation reactor is packed with the first hydrogenation catalyst in a larger proportion than the second hydrogenation catalyst. Thereby, the stability of the process for preparing neopentyl glycol may be enhanced by preventing the elution of catalyst components even in a high-temperature and high-pressure reaction. That is, since the process can be carried out for a long time, productivity can be increased. Furthermore, since a catalyst suitable for the location of the reactor is used, the method for preparing neopentyl glycol according to the present invention has a high yield of neopentyl glycol.

That is, the first hydrogenation catalyst according to the present invention is a copper-based catalyst.

In an exemplary embodiment of the present invention, the first hydrogenation catalyst may have a specific surface area of 200 $m^2/g$ or more, preferably 300 $m^2/g$ or more.

In an exemplary embodiment of the present invention, the first hydrogenation catalyst may have a specific surface area of 1,000 $m^2/g$ or less.

In an exemplary embodiment of the present invention, the first hydrogenation catalyst may have a specific surface area of 200 $m^2/g$ or more and 1,000 $m^2/g$ or less, preferably 300 $m^2/g$ or more and 1,000 $m^2/g$ or less.

In an exemplary embodiment of the present invention, the first hydrogenation catalyst may have a density of 500 g/L or more and less than 600 g/L, preferably 550 g/L or more and less than 600 g/L.

In general, the hydrogenation reaction activity tends to increase as the specific surface area of the hydrogenation catalyst increases. However, when the specific surface area of the hydrogenation catalyst increases, the density generally tends to decrease, which may cause the amount of silica (Si) eluted and the stability of the catalyst to increase and decrease, respectively. Since the first hydrogenation catalyst has high activity at low temperatures and simultaneously satisfies the above density range by satisfying the specific surface area range, the elution of silica may be prevented and the stability of the catalyst may be enhanced.

That is, the first hydrogenation catalyst is a catalyst having characteristics of exhibiting high activity at low temperatures while having an appropriate level of catalyst stability.

In an exemplary embodiment of the present invention, the first hydrogenation catalyst may be a catalyst with an extruded or spherical morphology.

In an exemplary embodiment of the present invention, the first hydrogenation catalyst may have a diameter of 1 mm to 6 mm, preferably 2 mm to 5 mm. When the diameter is satisfied, the yield of neopentyl glycol may be increased.

In an exemplary embodiment of the present invention, the second hydrogenation catalyst includes copper components, and may have a specific surface area of 30 $m^2$/g or more and less than 200 $m^2$/g and a density of 600 g/L or more. That is, the second hydrogenation catalyst may be a copper-based catalyst. However, the second hydrogenation catalyst has a different specific surface area and density range from the first hydrogenation catalyst.

In an exemplary embodiment of the present invention, the second hydrogenation catalyst may have a specific surface area of 30 $m^2$/g or more and less than 200 $m^2$/g, preferably 80 $m^2$/g or more and less than 160 $m^2$/g.

In an exemplary embodiment of the present invention, the second hydrogenation catalyst may have a density of 600 g/L or more, preferably 650 g/L or more.

In an exemplary embodiment of the present invention, the second hydrogenation catalyst may have a density of 800 g/L or less.

In an exemplary embodiment of the present invention, the second hydrogenation catalyst may have a density of 600 g/L or more and 800 g/L or less, and preferably 650 g/L or more and 800 g/L or less.

The second hydrogenation catalyst may minimize the decrease in the activity of the catalyst by satisfying the specific surface area range, and may reduce the amount of eluted silica (Si) by satisfying the density range, thereby increasing the stability of the catalyst. That is, since the second hydrogenation catalyst has high activity at low temperatures and simultaneously satisfies the above density range by satisfying the specific surface area range, the elution of silica may be prevented and the stability of the catalyst may be enhanced.

That is, the second hydrogenation catalyst is a catalyst having characteristics of being highly stable at high temperature and high pH while having an appropriate level of catalyst activity.

In an exemplary embodiment of the present invention, the second hydrogenation catalyst may be a catalyst with an extruded or spherical morphology.

In an exemplary embodiment of the present invention, the second hydrogenation catalyst may have a diameter of 2 mm to 8 mm, preferably 3 mm to 6 mm. When the diameter is satisfied, the yield of neopentyl glycol may be increased.

In an exemplary embodiment of the present invention, it is possible to further include packing the hydrogenation reactor with a hydrogenation catalyst different from the first hydrogenation catalyst and the second hydrogenation catalyst, such that a location between a location packed with the first hydrogenation catalyst and a location packed with the second hydrogenation catalyst in the hydrogenation reactor is packed with the hydrogenation catalyst.

An exemplary embodiment of the present invention provides a method for preparing neopentyl glycol, the method including: packing an inlet of a hydrogenation reactor with a first hydrogenation catalyst including copper; sequentially packing the hydrogenation reactor with a second hydrogenation catalyst and a third hydrogenation catalyst, such that an outlet of the hydrogenation reactor is packed with the third hydrogenation catalyst and a location between a location packed with the first hydrogenation catalyst and a location packed with the third hydrogenation catalyst is packed with the second hydrogenation catalyst; introducing a raw material gas including hydroxypivaldehyde and hydrogen into the hydrogenation reactor packed with the first hydrogenation catalyst to the third hydrogenation catalyst; and carrying out a hydrogenation reaction while sequentially bringing the introduced raw material gas into contact with the first hydrogenation catalyst to the third hydrogenation catalyst, in which the first hydrogenation catalyst to the third hydrogenation catalyst are different from each other and satisfy the following Equations 3 and 4.

$$[Equation\ 3]$$

$$x + y + z = 100 (\%)$$

$$[Equation\ 4]$$

$$x > y + z$$

In Equations 3 and 4,

x means the packing proportion (%) of the first hydrogenation catalyst in the hydrogenation reactor,
y means the packing proportion (%) of the second hydrogenation catalyst in the hydrogenation reactor, and
z means the packing proportion (%) of the third hydrogenation catalyst in the hydrogenation reactor.

The method for preparing neopentyl glycol according to the present invention may further include the third hydrogenation catalyst, and is characterized in that the reactor is also packed with the third hydrogenation catalyst in consideration of operating conditions and core performance. Even in this case, the reactor is packed with the first hydrogenation catalyst in the largest proportion, and the packing proportion of the first hydrogenation catalyst is higher than the total packing proportion of the second hydrogenation catalyst and the third hydrogenation catalyst.

Thereby, the stability of the process for preparing neopentyl glycol may be enhanced by preventing the elution of catalyst components even in a high-temperature and high-pressure reaction. That is, since the process may be carried out for a long time, productivity may be increased, and the yield of neopentyl glycol to be prepared is high.

When the first to third hydrogenation catalysts are used, the above-described description may be applied to the description on the first hydrogenation catalyst and the second hydrogenation catalyst.

In an exemplary embodiment of the present invention, the third hydrogenation catalyst includes copper components, and may have a specific surface area of 30 $m^2/g$ or more and less than 200 $m^2/g$ and a density of 600 g/L or more. That is, the third hydrogenation catalyst may be a copper-based catalyst. However, the third hydrogenation catalyst has a different specific surface area and density range from the first hydrogenation catalyst.

In an exemplary embodiment of the present invention, the third hydrogenation catalyst may have a specific surface area of 30 $m^2/g$ or more and less than 200 $m^2/g$, preferably 80 $m^2/g$ or more and less than 160 $m^2/g$.

In an exemplary embodiment of the present invention, the third hydrogenation catalyst may have a density of 600 g/L or more, preferably 650 g/L or more.

In an exemplary embodiment of the present invention, the third hydrogenation catalyst may have a density of 800 g/L or less.

In an exemplary embodiment of the present invention, the third hydrogenation catalyst may have a density of 600 g/L or more and 800 g/L or less, preferably 650 g/L or more and 800 g/L or less.

The third hydrogenation catalyst may minimize the decrease in the activity of the catalyst by satisfying the specific surface area range, and may reduce the amount of eluted silica (Si) by satisfying the density range, thereby increasing the stability of the catalyst. That is, since the third hydrogenation catalyst has high activity at low temperatures and simultaneously satisfies the above density range by satisfying the specific surface area range, the elution of silica may be prevented and the stability of the catalyst may be enhanced.

That is, like the second hydrogenation catalyst, the third hydrogenation catalyst is also a catalyst having characteristics of being highly stable at high temperature and high pH while having an appropriate level of catalyst activity.

In an exemplary embodiment of the present invention, the third hydrogenation catalyst may be a catalyst with an extruded or spherical morphology.

Here, the fact that a catalyst has a spherical morphology means that the catalyst is prepared by coating a spherical support with an active ingredient, unlike an extruded catalyst which is molded using an extruder.

In an exemplary embodiment of the present invention, the third hydrogenation catalyst may have a diameter of 2 mm to 8 mm, preferably 3 mm to 6 mm. When the diameter is satisfied, the yield of neopentyl glycol may be increased.

In an exemplary embodiment of the present invention, the specific surface area of the first to third hydrogenation catalysts may satisfy the following Equation 5.

$$[\text{Equation 5}]$$

$$S_a > S_b > S_c$$

In Equation 5,

$S_a$ is the specific surface area (unit: $m^2/g$) of the first hydrogenation catalyst,
$S_b$ is the specific surface area (unit: $m^2/g$) of the second hydrogenation catalyst, and
$S_c$ means the specific surface area (unit: $m^2/g$) of the third hydrogenation catalyst.

That is, the specific surface area of the catalyst packed at the inlet of the reactor is the largest, the specific surface area of the catalyst packed at the outlet of the reactor is the smallest, and the specific surface area of the catalyst packed at the position between the inlet and the outlet has an intermediate size.

In an exemplary embodiment of the present invention, the density of the first to third hydrogenation catalysts may satisfy the following Equation 6.

$$[\text{Equation 6}]$$

$$d_c > d_b > d_a$$

In Equation 6,

$d_a$ is the density (unit: g/L) of the first hydrogenation catalyst,
$d_b$ is the density (unit: g/L) of the second hydrogenation catalyst, and
$d_c$ means the density (unit: g/L) of the third hydrogenation catalyst.

That is, the density of the catalyst packed at the inlet of the reactor is the smallest, the density of the catalyst packed at the outlet of the reactor is the largest, and the density of the catalyst packed at the position between the inlet and the outlet has an intermediate size.

The method for preparing neopentyl glycol according to the present invention is characterized in that during the process of developing a catalyst in which the catalyst stability is enhanced and the catalyst activity is excellent due to excellent catalyst strength even under high-temperature and high-pressure conditions, the method and conditions of preparing the catalyst are optimized and applied. That is, in order to adjust the specific surface to a predetermined level or less and enhance the stability of the catalyst in a range that minimizes the decrease in the activity of the catalyst (or to reduce the amount of silica eluted), the yield of neopentyl glycol may be increased by applying a catalyst whose density has been adjusted to a predetermined level or more to a method for preparing neopentyl glycol.

That is, when the relationship between the specific surface area and density of the first to third hydrogenation catalysts satisfies Equation 5 and/or Equation 6 above, the activity and stability of the hydrogenation catalyst may be optimized, so that the case where neopentyl glycol is prepared using the same has an effect in which the yield of neopentyl glycol is excellent.

An exemplary embodiment of the present invention provides a method for preparing neopentyl glycol, the method including carrying out a hydrogenation reaction by introducing a hydroxypivaldehyde (HPA) solution and hydrogen into a hydrogenation reactor, in which the hydrogenation reactor is packed with a hydrogenation catalyst satisfying the characteristics of the above-described first to third hydrogenation catalysts.

That is, in an exemplary embodiment of the present invention, the hydrogenation reactor may be packed with the above-described first to third hydrogenation catalysts.

More specifically, in an exemplary embodiment of the present invention, the hydrogenation reactor may be packed with the above-described first and second hydrogenation catalysts, or the hydrogenation reactor may be packed with all of the above-described first to third hydrogenation catalysts.

In an exemplary embodiment of the present invention, the inlet and outlet of the hydrogenation reactor may be packed with alumina balls. The alumina balls may have a diameter of 2 mm to 6 mm and may function to uniformly disperse reactants.

In an exemplary embodiment of the present invention, the method for preparing neopentyl glycol may be a method known in the art, except for including the catalyst for preparing neopentyl glycol according to the present invention.

For example, the hydrogenation reactor may be a fixed bed reactor (FBR) packed with a catalyst for preparing neopentyl glycol, and in this case, the catalyst and the reaction product need not be separated, and the hydrogenation reactor is stably operated and economical because it is possible to lower the reaction temperature and reaction pressure compared to the related art, and has an effect of significantly reducing investment costs because the catalyst is easily replaced and the size of the reactor may be reduced.

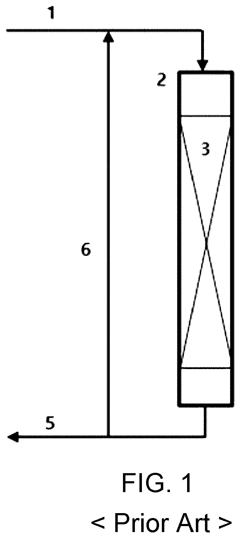

FIG. 1 shows an existing method for preparing neopentyl glycol, and exhibits a case where the reactor is packed with the first hydrogenation catalyst. Specifically, a reaction raw material supply pipe 1 which supplies a raw material gas including hydroxypivaldehyde and hydrogen is connected to a reactor 2, and the reactor 2 is packed with a first hydrogenation catalyst. A region 3 packed with the first hydrogenation catalyst in the reactor 2 is as shown in FIG. 1. In this case, the reactor 2 may be a fixed bed reactor (FBR) as described above. The raw material gas supplied from the reaction raw material supply pipe 1 is subjected to a hydrogenation reaction while being brought into contact with the first hydrogenation catalyst packed in the reactor 2, and as a result, neopentyl glycol may be obtained. The neopentyl glycol thus obtained may be conveyed from the reactor 2 through a reaction product outlet pipe 5. In this case, a part of neopentyl glycol may be transferred to the reaction raw material supply pipe 1 through a reaction product circulation pipe 6 and reused for the reaction in the reactor 2.

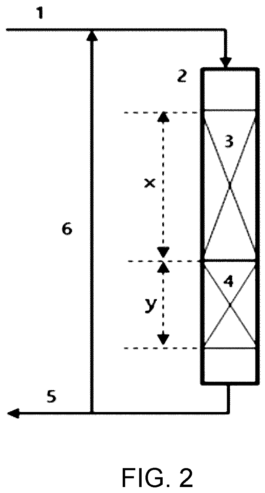

FIG. 2 shows a method for preparing neopentyl glycol according to the present invention, and exhibits a case where the reactor is packed with the first hydrogenation catalyst and the second hydrogenation catalyst. That is, compared to FIG. 1, FIG. 2 differs in that the inside of the reactor 2 is packed with the first hydrogenation catalyst and the second hydrogenation catalyst. Specifically, the inlet of the reactor 2 is packed with x% of the first hydrogenation catalyst, and the outlet of the reactor (2) is packed with y% of the second hydrogenation catalyst. A region 3 packed with the first hydrogenation catalyst and a region 4 packed with the second hydrogenation catalyst in the reactor 2 are as shown in FIG. 2. Furthermore, the inlet of the reactor 2 here means a location where the raw material gas is supplied. More specifically, the inlet of the reactor 2 means the location of the portion of the reactor 2 connected to the reaction raw material supply pipe 1.

The outlet of the reactor 2 means the location of the portion where the produced neopentyl glycol is discharged from the reactor. More specifically, the outlet of the reactor 2 means the location of the portion of the reactor 2 connected to the reaction product outlet pipe 5.

In this case, the packing proportion of the first hydrogenation catalyst is higher than the packing proportion of the second hydrogenation catalyst. That is, as shown in FIG. 2, the conditions of x + y = 100 and x > y are satisfied. In addition, the description on FIG. 1 may be applied to FIG. 2.

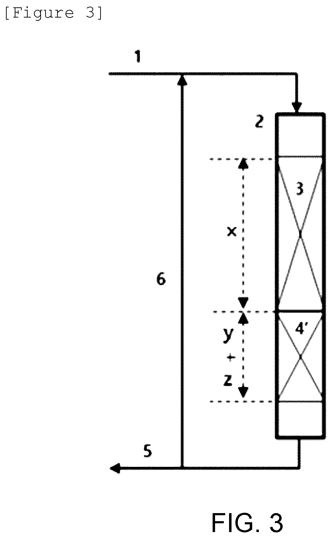

FIG. 3 shows another method for preparing neopentyl glycol according to the present invention, and exhibits a case where the reactor is packed with the first to third hydrogenation catalysts. In this case, the packing proportion of the first hydrogenation catalyst is higher than the sum of the packing proportion of the second hydrogenation catalyst and the packing proportion of the third hydrogenation catalyst. That is, as shown in FIG. 3, the conditions of x + y + z = 100 and x > y + z are satisfied. In addition, the description on FIGS. 1 and 2 may be applied to FIG. 3. In this case, a region 3 packed with the first hydrogenation catalyst and a region 4' packed with the second hydrogenation catalyst and the third hydrogenation catalyst in the reactor 2 are as shown in FIG. 3.

In an exemplary embodiment of the present invention, the hydroxypivaldehyde solution may include 50 wt% to 80 wt% of hydroxypivaldehyde, 1 wt% to 5 wt% of neopentyl glycol, 15 wt% to 35 wt% of alcohol, and 1 wt% to 10 wt% of water, and in this case, there is an effect of suppressing the production of by-products because the heat of reaction may be minimized without reducing the reactivity. Further, the hydroxypivaldehyde solution may further include a high-boiling point material. That is, in an exemplary embodiment of the present invention, the hydroxypivaldehyde solution may further include 1 wt% to 6 wt% of the high-boiling point material.

Furthermore, in an exemplary embodiment of the present invention, the hydrogenation reaction may be performed at a reaction temperature of 100°C to 250°C, preferably 100°C to 200°C, and more preferably 100°C to 180°C.

Further, in an exemplary embodiment of the present invention, the hydrogenation reaction may be performed at a reaction pressure of 35 bar or more. The reaction pressure means the measured pressure.

When the catalyst for preparing neopentyl glycol according to an exemplary embodiment of the present invention is applied to the preparation of neopentyl glycol, it is possible to prevent a problem in that catalyst components (for example, silica components) in a neopentyl glycol solution to be prepared are eluted.

Therefore, when the catalyst for preparing neopentyl glycol according to an exemplary embodiment of the present invention is applied to the preparation of neopentyl glycol, there is no need for a purification process due to the elution of catalyst components (for example, silica components), and the catalyst life is extended, so that it is possible to obtain an effect capable of lowering preparation costs.

### Examples

Hereinafter, the present invention will be described in detail with reference to Examples for specifically describing the present invention. However, the Examples according to the present invention may be modified in various forms, and it is not interpreted that the scope of the present invention is limited to the Examples to be described in detail below. The Examples of the present invention are provided for more completely explaining the present invention to the person with ordinary skill in the art.

### <Experimental Example 1> Measurement of performance of hydrogenation catalyst

The performance of the hydrogenation catalyst was measured using the hydrogenation catalyst A to catalyst C, which are $CuO/SiO_2$-based hydrogenation catalysts, a reaction raw material composed of 65 wt% of HPA, 2 wt% of NPG, 25 wt% of 2-ethyl hexanol (2-EH), 5 wt% of $H_2O$, and 3 wt% of a high-boiling point material.

First, in order to measure the activity of the catalyst, 2.5 ml of Catalyst A to Catalyst C was added to 100 g of the reaction raw material, and then the amount of $H_2$ consumed was measured while performing a hydrogenation reaction under the conditions of 160°C and 35 bar for 1 hour (hr). When the amount of $H_2$ consumed is large, it means that the activity of the catalyst is high.

Further, in order to measure the amount of Si eluted from the catalyst support, after a small amount of each of Catalysts A to C was added to an excess amount of the reaction raw material, a hydrogenation reaction was carried out for 6 hours at 180°C, which is a harsher condition than normal reaction conditions, and then the content of Si eluted in the reaction product was measured. The Si content was measured by ICP-OES analysis. More specifically, the content of Si eluted into the reaction product after the hydrogenation reaction compared to the content of Si included in the initial catalyst was calculated as a relative ratio. A high content of Si eluted means that the stability of the catalyst is relatively low.

Further, the specific surface area, density, and diameter of each of the hydrogenation catalysts A to C were measured using the BET method, a 1 L measuring cylinder, and vernier calipers.

The characteristics of hydrogenation catalysts A to C measured by the above method are as shown in the following Table 1.

[Table 1]

| Hydrogenation Catalyst | -based | Specific surface area ($m^2/g$) | Density (g/L) | Diameter (mm) | Amount of $H_2$ consumed (ml/min. Cat.ml) | Catalyst activity (%) | Si Elution amount (%) |
|---|---|---|---|---|---|---|---|
| Catalyst A (extruded) | $CuO/SiO_2$ | 360 | 570 | 4 | 15.0 | 100 | 27 |
| Catalyst B (extruded) | $CuO/SiO_2$ | 130 | 698 | 5 | 13.4 | 89 | 5 |
| Catalyst C (spherical) | $CuO/SiO_2$ | 100 | 720 | 3 | 13.0 | 87 | 3 |
| Catalyst D | Ni | - | - | - | - | - | - |

In the following Examples and Comparative Examples, Catalyst A to Catalyst C whose performance was measured in Experimental Example 1 above were used as hydrogenation catalysts. More specifically, Catalysts A and B are catalysts with an extruded morphology, and Catalyst C corresponds to a catalyst with a spherical morphology. For reference, as Catalyst A, a commercial catalyst called Octolyst 2009 product manufactured by Evonik Industries was used, and as Catalyst D, a NiSAT360 product manufactured by Clariant was used.

In addition, Catalyst B was prepared by synthesizing a catalyst intermediate using a copper precursor and an aqueous silica sol solution, and then pulverizing the catalyst intermediate into a fine powder. Thereafter, a known binder was mixed with Catalyst B to extrude the catalyst, and then the extruded catalyst was dried at a temperature of 100°C or lower. Thereafter, Catalyst B was prepared by performing firing at a temperature of 250°C to 450°C.

Furthermore, Catalyst C was prepared by preparing a coating powder using a copper precursor and an aqueous silica sol solution, coating a support with the coating powder using an aqueous alcohol solution, and then drying and firing the coated support. Here, the coprecipitation, drying and firing steps were carried out in the same manner as the conditions for Catalyst C.

Further, the catalyst activity and elution amount were calculated using the following equations.

* Catalyst activity (%) = amount of $H_2$ consumed of target catalyst (L)/amount of $H_2$ consumed of reference catalyst (L) * 100 (%) * Amount of Si eluted (%) = content of Si eluted into reaction product after reaction (parts by weight) / total amount of Si included in initial catalyst (parts by weight) $\times$ 100 (%)

Catalyst A was defined as a reference catalyst in the catalyst activity.

## 1) Examples

A fixed bed reactor with a reactor diameter of 35.5 mm was used to pack the top of the reactor with 3 mm and 5 mm alumina balls at a height of 100 mm, respectively, for uniform distribution of reactants.

Subsequently, the reactor was sequentially packed with the hydrogenation catalyst A and Catalyst B to a height of 350 mm and 200 mm, respectively, from below. The bottom of the reactor was packed with 3 mm alumina balls to 200 mm.

While supplying 2-EH at a flow rate of 1.7 g/min and $H_2$ at a flow rate of 100 ml/min to the reactor packed with the catalyst, catalyst activation was first performed by increasing the temperature of the reactor step by step from 120°C to 200°C.

Once the catalyst activation was completed, a hydrogenation reaction was started while supplying a reaction raw material composed of 65 wt% of HPA, 2 wt% of NPG, 25 wt% of 2-EH, 5 wt% of $H_2O$, and 3 wt% of a high-boiling point material at a constant flow rate of 3.4 g/min. In this case, while maintaining the inlet temperature of the catalyst layer in the reactor at 130°C, the hydrogenation reaction product was recirculated to the inlet of the reactor at a flow rate of 17 g/min in order to control the temperature of the catalyst layer caused by the heat of hydrogenation reaction not to exceed 180°C.

While continuous operation was performed for 72 hours, the HPA conversion rate and NPG yield were calculated by confirming the composition of the reaction product by GC analysis, and the total amount of Si eluted from the reaction product was confirmed by ICP-OES analysis. For GC analysis, organic materials were analyzed using an HP-1 (L: 30 m, ID: 0.32 mm, film: 1.05 m) column, and using a gas chromatography analysis device (device name: Agilent 7890) under the temperature conditions of 70°C/3 min-10°C/min-280°C/35 min of an oven, and in this case, the organic material analysis was measured using FID as a detector at a temperature of 280°C.

ICP-OES analysis was performed under the same conditions as Experimental Example 1.

The results are shown in the following Table 2.

## 2) Examples 2 and 3 and Comparative Examples 1 and 2

The hydrogenation reactions of Examples 2 and 3 and Comparative Examples 1 and 2 were performed in the same manner as in Example 1, except that the top, middle, and bottom of the catalyst layer were packed with the catalysts shown in Table 1 above to the heights shown in the following Table 2, and then the HPA conversion rate and NPG yield were calculated, and the total amount of Si eluted was confirmed.

The results are shown in the following Table 2.

## 3) Comparative Examples 3 to 5

The hydrogenation reactions of Comparative Examples 3 to 5 were performed in the same manner as in Example 1, except that the top, middle, and bottom of the catalyst layer were packed with the catalysts shown in Table 1 above to the heights shown in the following Table 2, and then the HPA conversion rate and NPG yield were calculated, and the amount of Si eluted was confirmed. The results are shown in the following Table 2.

[Table 2]

| No. | Type and packing height (mm) of catalyst | | | HPA conversion rate (%) | NPG yield (%) | Amount of Si eluted (%) |
|---|---|---|---|---|---|---|
| | Top | Middle | Bottom | | | |
| Example 1 | A (350) | - | B (200) | 100 | 99 | 0.06 |
| Example 2 | A (350) | - | C (200) | 100 | 99 | 0.06 |
| Example 3 | A (350) | B (100) | C (100) | 100 | 99 | 0.07 |
| Comparative Example 1 | A (550) | - | - | 100 | 99 | 0.3 |
| Comparative Example 2 | B (550) | - | - | 98 | 97 | 0.05 |
| Comparative Example 3 | B (350) | - | C (200) | 97.4 | 96.5 | 0.07 |
| Comparative Example 4 | A (150) | B (300) | C (100) | 98.5 | 98 | 0.06 |
| Comparative Example 5 | D (550) | | | Not measurable | Not measurable | Not measurable |

In Table 2 above, Examples 1 and 2 mean that the reactor is packed with two types of catalysts corresponding to Catalyst A and Catalyst B and Catalyst A and Catalyst C, respectively, and Example 3 means that the reactor is packed with three types of catalysts of Catalysts A to C, and Comparative Examples 1 and 2 mean that the reactor is packed with one type of catalyst in opposition to Catalyst A and Catalyst B, respectively. The numbers in parentheses mean the packing height of the packed catalyst. In addition, the conversion rate, yield, and elution amount were calculated using the following equations.

* HPA conversion rate (%) = number of moles of reacted HPA/number of moles of supplied HPA $\times$ 100 (%)

* NPG yield = number of moles of produced NPG/number of moles of supplied HPA $\times$ 100 (%)

* Amount of Si eluted (%) = content of Si eluted into reaction product after reaction (parts by weight)/total amount of Si included in initial catalyst (parts by weight) $\times$ 100 (%)

As can be seen from the results in Table 2 above, it could be confirmed that when a single catalyst is used in the process of packing the reactor with the hydrogenation catalyst as in Comparative Examples 1 and 2, or when a copper-containing first hydrogenation catalyst packed in the top has a specific surface area of less than 200 m$^2$/g and a density of 600 g/L or more as in Comparative Example 3, the amount of Si eluted is relatively high or the NPG yield is reduced, whereas when a mixture of two or more types of hydrogenation catalysts is used for packing as in Examples 1 to 3, the NPG yield is as high as 99%, and the amount of Si eluted is also reduced.

Specifically, as in Comparative Example 1, when Catalyst A, which satisfies the conditions of a specific surface area of 200 m$^2$/g or more and a density of 500 g/L or more and less than 600 g/L, was used as the copper-containing first hydrogenation catalyst introduced into the top, the amount of Si eluted was 0.3% even though the HPA conversion rate and NPG yield were good, which caused the life of the catalyst to be shortened due to the decrease in the strength of the catalyst, and as in Comparative Example 2, when Catalyst B, which does not satisfy the specific surface area of 200 m$^2$/g or more and the density of 500 g/L or more and less than 600 g/L, was used as the copper-containing first hydrogenation catalyst introduced into the top, the HPA conversion rate and NPG yield each fell below 99% even though the amount of Si eluted was good. In particular, when the HPA conversion rate is less than 99%, there is a problem in that unreacted materials accumulate in the reaction product circulation pipe 6, resulting in a remarkable increase in the amount of catalyst required. As in Comparative Example 3, Catalyst C, which had a specific surface area of 30 m$^2$/g or more and less than 200 m$^2$/g and a density of 600 g/L or more, was combined in the bottom while using Catalyst B in the top, but as in Comparative Example 2, no improvement in HPA conversion rate and NPG yield appeared.

Comparative Example 4 corresponds to a case where [Equation 2] is not satisfied by packing the top with Catalyst A having a specific surface area of 200 m$^2$/g or more and a density of 500 g/L or more and less than 600 g/L to a height of 150 mm, packing the middle with Catalyst B having a specific surface area of 30 m$^2$/g or more and less than 200 m$^2$/g and a density of 600 g/L or more to a height of 300 mm, and packing the bottom with Catalyst C having a specific surface area of 30 m$^2$/g or more and less than 200 m$^2$/g and a density of 600 g/L or more to a height of 100 mm, and even though the amount of Si eluted was maintained at a good level, the HPA conversion rate and NPG yield were each at a level less than 99%, and no improvement in the HPA conversion rate and NPG yield appeared.

Furthermore, in the case of Comparative Example 5, the HPA hydrogenation reaction was performed by introducing a Ni-based catalyst instead of copper into the top of the catalyst layer, but while the continuous reaction was performed for 72 hours, and the catalyst activity was continuously decreased, making it impossible to measure stable catalyst activity. In other words, the reaction stability of the Ni-based catalyst was so low that the catalyst activity for the HPA hydrogenation reaction could not be measured, and thus it was found that the catalyst was not suitable as a catalyst for preparing NPG.

That is, it could be confirmed that when the method for preparing neopentyl glycol according to the present invention is applied, the decrease in the strength of the catalyst can be suppressed and the life of the catalyst can be extended by reducing the elution amount of Si component, which is a catalyst support, while maintaining a high reaction yield.

**Claims**

1. A method for preparing neopentyl glycol, the method comprising:

packing an inlet of a hydrogenation reactor with a first hydrogenation catalyst comprising copper;
packing an outlet of the hydrogenation reactor with a second hydrogenation catalyst;
introducing a raw material gas comprising hydroxypivaldehyde and hydrogen into the hydrogenation reactor packed with the first hydrogenation catalyst and the second hydrogenation catalyst; and
carrying out a hydrogenation reaction while sequentially bringing the introduced raw material gas into contact with the first hydrogenation catalyst and the second hydrogenation catalyst,
wherein the first hydrogenation catalyst has a specific surface area of 200 m$^2$/g or more and a density of 500 g/L or more and less than 600 g/L, and
the first hydrogenation catalyst and the second hydrogenation catalyst are different from each other and satisfy the following Equations 1 and 2:

$$[Equation\ 1]$$

$$x + y = 100(\%)$$

[Equation 2]

$$x > y$$

in Equations 1 and 2:
x means the packing proportion (%) of the first hydrogenation catalyst in the hydrogenation reactor, and

y means the packing proportion (%) of the second hydrogenation catalyst in the hydrogenation reactor.

2. The method of claim 1, wherein the first hydrogenation catalyst has a diameter of 1 mm to 6 mm.

3. The method of claim 1, wherein the second hydrogenation catalyst comprises a copper component, and the second hydrogenation catalyst has a specific surface area of 30 m$^2$/g or more and less than 200 m$^2$/g and a density of 600 g/L or more.

4. The method of claim 1, wherein the second hydrogenation catalyst has a diameter of 2 mm to 8 mm.

5. A method for preparing neopentyl glycol, the method comprising:

packing an inlet of a hydrogenation reactor with a first hydrogenation catalyst comprising copper;
sequentially packing the hydrogenation reactor with a second hydrogenation catalyst and a third hydrogenation catalyst, such that an outlet of the hydrogenation reactor is packed with the third hydrogenation catalyst and a location between a location packed with the first hydrogenation catalyst and a location packed with the third hydrogenation catalyst is packed with the second hydrogenation catalyst;
introducing a raw material gas comprising hydroxypivaldehyde and hydrogen into the hydrogenation reactor packed with the first hydrogenation catalyst to the third hydrogenation catalyst; and
carrying out a hydrogenation reaction while sequentially bringing the introduced raw material gas into contact with the first hydrogenation catalyst to the third hydrogenation catalyst,
the first hydrogenation catalyst to the third hydrogenation catalyst are different from each other and satisfy the following Equations 3 and 4:

[Equation 3]

$$x + y + z = 100 (\%)$$

[Equation 4]

$$x > y + z$$

in Equations 3 and 4:

x means the packing proportion (%) of the first hydrogenation catalyst in the hydrogenation reactor,
y means the packing proportion (%) of the second hydrogenation catalyst in the hydrogenation reactor, and
z means the packing proportion (%) of the third hydrogenation catalyst in the hydrogenation reactor.

6. The method of claim 5, wherein the third hydrogenation catalyst comprises a copper component, and the third hydrogenation catalyst has a specific surface area of 30 m$^2$/g or more and less than 200 m$^2$/g and a density of 600 g/L or more.

7. The method of claim 5, wherein the third hydrogenation catalyst has a diameter of 2 mm to 8 mm.

8. The method of claim 5, wherein the specific surface areas of the first hydrogenation catalyst to the third hydrogenation catalyst satisfy the following Equation 5:

[Equation 5]

$$S_a > S_b > S_c$$

in Equation 5:

$S_a$ is the specific surface area (unit: $m^2/g$) of the first hydrogenation catalyst,
$S_b$ is the specific surface area (unit: $m^2/g$) of the second hydrogenation catalyst, and
$S_c$ means the specific surface area (unit: $m^2/g$) of the third hydrogenation catalyst.

9. The method of claim 5, wherein the density of the first hydrogenation catalyst to the third hydrogenation catalyst satisfies the following Equation 6:

[Equation 6]

$$d_c > d_b > d_a$$

in Equation 6:

$d_a$ is the density (unit: g/L) of the first hydrogenation catalyst,
$d_b$ is the density (unit: g/L) of the second hydrogenation catalyst, and
$d_c$ means the density (unit: g/L) of the third hydrogenation catalyst.

[Equation 5]

$$S_a > S_b > S_c$$

FIG. 1

< Prior Art >

FIG. 2

[Figure 3]

FIG. 3

## INTERNATIONAL SEARCH REPORT

| International application No. |
| --- |
| **PCT/KR2024/001979** |

| A. | CLASSIFICATION OF SUBJECT MATTER |
| --- | --- |

**C07C 29/141**(2006.01)i; **C07C 29/94**(2006.01)i; **C07C 31/20**(2006.01)i; **B01J 23/72**(2006.01)i

According to International Patent Classification (IPC) or to both national classification and IPC

| B. | FIELDS SEARCHED |
| --- | --- |

Minimum documentation searched (classification system followed by classification symbols)

C07C 29/141(2006.01); B01J 21/08(2006.01); B01J 23/72(2006.01); B01J 23/755(2006.01); B01J 23/89(2006.01); B01J 31/08(2006.01); C07C 29/145(2006.01); C07C 31/20(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 네오펜틸글리콜(neopentyl glycol), 촉매(catalyst), 수소화 반응(hydrogenation), 표면적(surface area), 밀도(density)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| DY | KR 10-2011-0038324 A (LG CHEM, LTD.) 14 April 2011 (2011-04-14)<br>    See claims 1 and 3; and paragraphs [0018], [0019] and [0041]. | 1-9 |
| Y | KR 10-2018-0035583 A (LOTTE CHEMICAL CORPORATION) 06 April 2018 (2018-04-06)<br>    See claim 2; and paragraphs [0002] and [0025]. | 1-9 |
| A | CN 115069268 A (SHANDONG LUXIN DESIGN ENGINEERING CO., LTD.) 20 September 2022 (2022-09-20)<br>    See entire document. | 1-9 |
| A | KR 10-2052084 B1 (LG CHEM, LTD.) 04 December 2019 (2019-12-04)<br>    See entire document. | 1-9 |
| A | KR 10-2013-0064752 A (BASF SE) 18 June 2013 (2013-06-18)<br>    See entire document. | 1-9 |

| ☐ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **30 May 2024** | **31 May 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/001979**

| Patent document cited in search report | | | Publication date (day/month/year) | Patent family member(s) | | | Publication date (day/month/year) |
|---|---|---|---|---|---|---|---|
| KR | 10-2011-0038324 | A | 14 April 2011 | KR | 10-1219258 | B1 | 10 January 2013 |
| KR | 10-2018-0035583 | A | 06 April 2018 | KR | 10-1860148 | B1 | 21 May 2018 |
| CN | 115069268 | A | 20 September 2022 | None | | | |
| KR | 10-2052084 | B1 | 04 December 2019 | KR | 10-2017-0034513 | A | 29 March 2017 |
| KR | 10-2013-0064752 | A | 18 June 2013 | BR | 112012028567 | A2 | 02 August 2016 |
| | | | | CN | 102884032 | A | 16 January 2013 |
| | | | | EP | 2569271 | A1 | 20 March 2013 |
| | | | | EP | 2569271 | B1 | 14 May 2014 |
| | | | | ES | 2476341 | T3 | 14 July 2014 |
| | | | | JP | 2013-526506 | A | 24 June 2013 |
| | | | | RU | 2012153379 | A | 20 June 2014 |
| | | | | SG | 185512 | A1 | 28 December 2012 |
| | | | | TW | 201200494 | A | 01 January 2012 |
| | | | | WO | 2011-141470 | A1 | 17 November 2011 |

Form PCT/ISA/210 (patent family annex) (July 2022)

**EP 4 667 448 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 2024001979 W
- KR 1020230018881
- KR 1020240019690
- KR 1020110038324